# EUROPEAN PATENT APPLICATION

(11) **EP 2 977 974 A1**
(43) Date of publication of application: **27.01.2016**
(21) Application number: 14768450.0
(22) Date of filing: 18.03.2014
(51) Int. Cl.: G08B 25/04, A61B 5/00, G08B 21/04, G08B 23/00

(54) **LIFESTYLE CARE ASSISTING DEVICE**

(30) Priority: 21.03.2013 JP 2013059165
(71) Applicant: Kabushiki Kaisha Toshiba, Inc., Tokyo 105-8001 (JP); Toshiba Solutions Corporation, Kawasaki-shi, Kanagawa 212-8585 (JP)
(72) Inventor: OKAMOTO, Toshio, Kawasaki-shi Kanagawa 212-8585 (JP); TAKAYAMA, Takuzo, Tokyo 105-8001 (JP); UENO, Ken, Tokyo 105-8001 (JP); SUZUKI, Takuji, Tokyo 105-8001 (JP)
(74) Representative: Hoffmann Eitle
(86) International application number: PCT/JP2014/001526
(87) International publication number: WO 2014/148037

(57) **Abstract**

[PROBLEM] To obtain the health condition of a user through measurement and determination of physiological information based on a living behavior situation of the user and providing the obtained health condition for a watcher.

[SOLVING MEANS] A life watching and support apparatus of an embodiment is connected to a living behavior acquirement apparatus configured to acquire behavior information of a user, to a physiological information acquirement apparatus configured to acquire physiological information of the user, and to a display apparatus on a watcher side watching the user. The life watching and support apparatus includes a behavior estimation section configured to estimate a living behavior situation of the user based on the behavior information of the user, a health condition determination section configured to determine a health condition of the user based on the estimated living behavior situation and the physiological information, and an output control section configured to display a watching screen including the determined health condition in the display apparatus on the watcher side depending on the health condition.

## Description

### TECHNICAL FIELD

The present invention relates to a life watching and support apparatus capable of measuring and determining various states of a user to allow a watcher such as a family member to know the health condition of the user.

### BACKGROUND ART

Technologies have conventionally been proposed which utilize a biometric sensor apparatus wearable by a user during use to obtain the health condition from physiological information of the user. Technologies have also been proposed for monitoring living behaviors of a user to obtain the health condition from changes in the living behaviors.

### PRIOR ART DOCUMENTS

### PATENT DOCUMENTS

[Patent Document 1] Japanese Patent No. 3846844
[Paten Document 2] Japanese Patent Laid-Open No. 2004-313461

### DISCLOSURE OF THE INVENTION

### PROBLEMS TO BE SOLVED BY THE INVENTION

It is an object of the present invention to provide a life watching and support apparatus capable of obtaining the health condition of a user through measurement and determination of physiological information based on a living behavior situation of the user and providing the obtained health condition for a watcher.

### MEANS FOR SOLVING THE PROBLEMS

A life watching and support apparatus of an embodiment is connected to a living behavior acquirement apparatus configured to acquire behavior information of a user, to a physiological information acquirement apparatus configured to acquire physiological information of the user, and to a display apparatus on a watcher side watching the user. The life watching and support apparatus includes a behavior estimation section configured to estimate a living behavior situation of the user based on the behavior information of the user, a health condition determination section configured to determine a health condition of the user based on the estimated living behavior situation and the physiological information, and an output control section configured to display a watching screen including the determined health condition in the display apparatus on the watcher side depending on the health condition.

### BRIEF DESCRIPTION OF THE DRAWINGS

[Fig. 1] A diagram showing an exemplary system configuration of a life watching and support system according to Embodiment 1.
[Fig. 2] A block diagram showing exemplary components of a life watching and support apparatus, a physiological information measurement apparatus, and a living behavior acquirement apparatus according to Embodiment 1.
[Fig. 3] A table showing an example of a rule of displaying watching screens displayed in a display apparatus on a watcher side and a rule of notifying watchers depending on health conditions according to Embodiment 1.
[Fig. 4] A table showing an example of contact information of watchers according to Embodiment 1.
[Fig. 5] A diagram showing an example of a watching screen displayed in the display apparatus on the watcher side according to Embodiment 1 and representing an exemplary watching screen for normal when the health condition is normal.
[Fig. 6] A table showing an example of a living behavior situation list according to Embodiment 1.
[Fig. 7] A table showing an example of a health situation information screen according to Embodiment 1.
[Fig. 8] A diagram showing an example of physiological situation information according to Embodiment 1.
[Fig. 9] A diagram showing an example of the watching screen displayed in the display apparatus on the watcher side according to Embodiment 1 and representing an exemplary watching screen for non-normal when the health condition is non-normal.
[Fig. 10] A diagram showing an example of the watching screen displayed in the display apparatus on the watcher side according to Embodiment 1 and representing an exemplary watching screen for abnormal when the health condition is abnormal.
[Fig. 11] A diagram showing an example of an emergency screen displayed in a display apparatus on a user side according to Embodiment 1.
[Fig. 12] A diagram showing an example of a health monitor screen (for non-normal) displayed in the display apparatus on the user side according to Embodiment 1.
[Fig. 13] A diagram showing an example of the health monitor screen (for normal) displayed in the display apparatus on the user side according to Embodiment 1.
[Fig. 14] A diagram showing a flow of processing in the life watching and support system according to Embodiment 1.
[Fig. 15] A diagram for describing Example 1 of a life watching aspect using the life watching and support system.
[Fig. 16] A diagram for describing Example 2 of the life watching aspect using the life watching and support system.
[Fig. 17] A diagram for describing a modification of Example 2 shown in Fig. 16.

### MODE FOR CARRYING OUT THE INVENTION

A preferred embodiment will hereinafter be described with reference to the drawings.

### EMBODIMENT 1

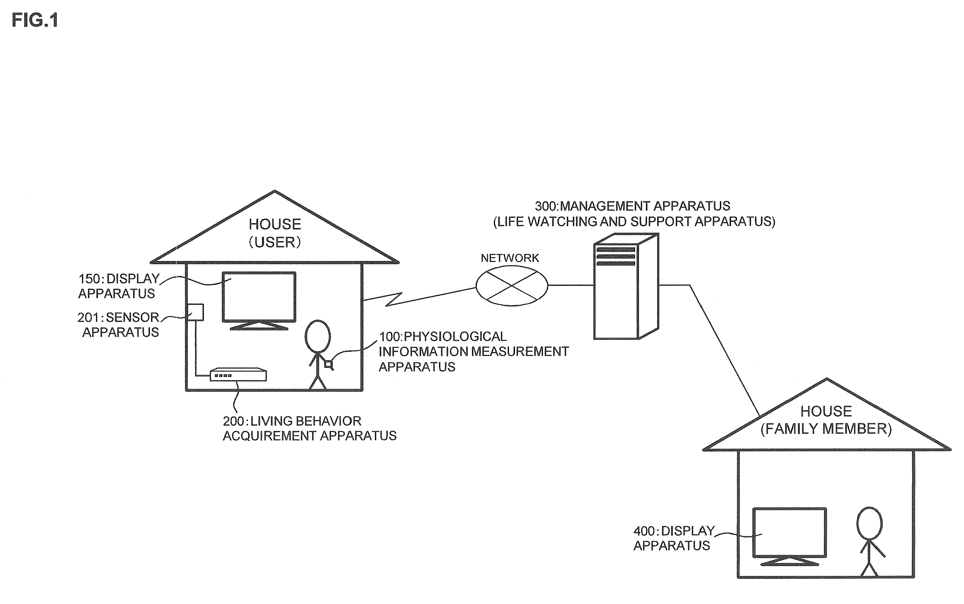

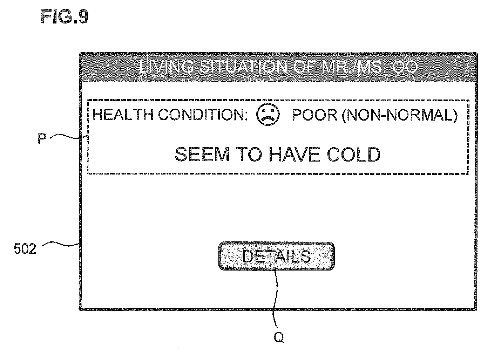

Fig. 1 to Fig. 17 are diagrams showing a life watching and support system according to Embodiment 1. The life watching and support system of the present embodiment is a computer system for allowing a user such as an elderly person and a live-alone to be watched by a watcher such as a user's family member, relative, or a public agency (such as a medical agency and a care agency), and obtains the daily health condition of the user from his/her living behavior situation and physiological information and provides the obtained health condition for the watcher.

As shown in Fig. 1, the life watching and support system includes a physiological information measurement apparatus 100 for acquiring physiological information of a user (person to be watched) such as an elderly person, a living behavior acquirement apparatus 200 provided with a detection apparatus 201 usually installed in a house of the user for primarily acquiring information about behaviors of the user in the house, and a management apparatus (life watching and support apparatus) 300 for receiving the physiological information and the behavior information from the physiological information measurement apparatus 100 and the living behavior acquirement apparatus, respectively, over a network and obtaining the user's health condition.

The management apparatus 300 is connected over a network to a display apparatus 400 installed in a house of a watcher on a watching side and displays the obtained health condition of the user in the display apparatus 400 to allow a family member or the like on the watcher side to visually know the user's health condition. The management apparatus 300 is also connected to a display apparatus 150 usually installed in the user's house to visually provide the obtained health condition for the user to be watched basically.

The display apparatuses of the watcher and the user for displaying the user's health condition are not limited to display apparatuses installed in their houses and can be any apparatus capable of communication with the management apparatus 300 including a portable terminal and a telephone with a monitor allowing the visual presentation of the health condition in which the user's health condition can be obtained outside the house. Instead of the display apparatus, an apparatus capable of outputting audio can be used. In this case, the management apparatus 300 can have the function of converting the health condition into audio information to perform the audio output of the health condition.

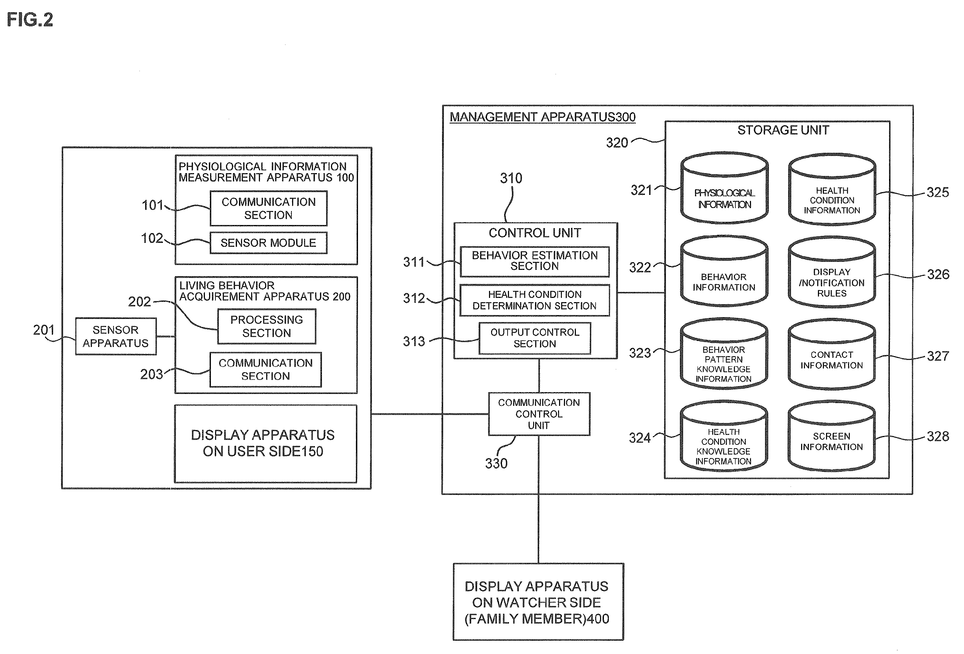

As shown in Fig. 2, the physiological information measurement apparatus 100 is a wearable biometric sensor put on the user's body to measure the physiological information. Examples of the physiological information measurement apparatus include a head-mount type and a wrist type put on a user's wrist, and a known physiological information measurement apparatus can be used. The physiological information measurement apparatus 100 includes a communication section 101 for controlling communication such as wireless communication and infrared communication and can communicate data with the management apparatus 300, another communication device installed in the house connected to the management apparatus 300, and a cellular phone.

Even when the user wearing the physiological information measurement apparatus 100 is outside the house, the communication section 101 for controlling communication such as wireless communication and infrared communication enables data communication with the management apparatus 300 and another communication device installed in the house connected to the management apparatus 300. When the user carries a cellular phone having the GPS function, the user can be located, and life information outside the house can be acquired.

The physiological information measurement apparatus 100 includes a plurality of sensor modules 102. The sensor modules are sensor apparatuses for measuring various types of physiological information including the heart rate, pulse, blood pressure (pulse wave), and body temperature. Each sensor module can be attached to part of the body such as a fingertip, chest, and head to measure the physiological information including the pulse, heart rate, Galvanic Skin Response (GSR), body temperature (body surface temperature), blood pressure, and electrocardiogram. The apparatus 100 can also include a behavior information acquiring function, later described, provided by an acceleration sensor, pedometer, sound-collecting microphone and the like.

The physiological information measurement apparatus 100 does not necessarily include the sensor modules integrated into one unit for measuring all types of physiological information, and a plurality of apparatuses may be provided for the respective measurement functions (sensor modules). Thus, a plurality of physiological information measurement apparatuses can measure a plurality of types of physiological information, or a single physiological information measurement apparatus can measure a plurality of types of physiological information. Of the plurality of types of physiological information, the pulse and the body temperature may be measured by a single sensor module, for example. A single sensor module may measure a single or a plurality of types of physiological information.

The physiological information measurement apparatus 100 can automatically start the measurement of physiological information by sensing the user putting on the physiological information measurement apparatus 100. For example, sensing of a bioelectric signal or body temperature can trigger the start of the measurement. The measurement of physiological information may be performed at regular time intervals or at a predetermined point in time in response to a particular living behavior situation estimated from behavior information, for example.

The physiological information measurement apparatus 100 can include not only the wearable biometric sensor wearable on the user's body but also a stationary physiological information measurement apparatus fixed in the user's house. Examples thereof include a manometer and a weight scale. In this case, the manometer or the weight scale can be provided with a communication function to allow data communication with the management apparatus 300 via another communication device installed in the house connected to the management apparatus 300 or the wearable physiological information measurement apparatus 100.

The stationary physiological information measurement apparatus 100 may be used by a plurality of users living in the same house. Thus, physiological information of a user can be transmitted to the management apparatus 300 together with authentication information such as the user's ID to manage measured physiological information for each user. The stationary physiological information measurement apparatus such as the manometer and the weight scale may transmit the physiological information together with authentication information such as the user's ID directly to the management apparatus 300 by using the communication function. The communication may also be performed over a medium such as a cellular phone capable of communication with the management apparatus 300 or the Internet (for example, with Wi-Fi(R)), although such a medium is not installed in the house connected to the management apparatus 300.

The physiological information measured by the physiological information measurement apparatus 100 can be associated with a measurement time to acquire the physiological information in temporal sequence at predetermined points in time. In this case, the physiological information does not need to be transmitted in real time, so that the amount of data communication can be limited.

As shown in Fig. 2, the living behavior acquirement apparatus 200 is connected to the sensor apparatus 201 installed in the user's house such as a human detection sensor, infrared sensor, pressure sensor, temperature sensor, camera (imaging apparatus), and sound-collecting microphone, and acquires, as behavior information, various types of information detected by the sensor apparatus 201 that correspond to the behaviors of the user. In the living behavior acquirement apparatus 200, a processing section 202 processes the detection information output from the sensor apparatus 201, outputs the behavior information of the user in temporal sequence, and transmits the information to the management apparatus 300 via a communication section 203. The behavior information can be acquired at any time in response to sensing by the sensor apparatus 201, or at a predetermined point in time in association with another sensor. For example, video or audio can be started to be collected at the time when the human detection sensor detects the user.

The living behavior acquirement apparatus 200 can be connected to an electrical appliance such as an air conditioner, heater, washing machine, and television apparatus in the user's house, or an electrical/gas appliance such as a wattmeter, water heater, IH cooking heater, and gas cooking stove, and can detect, as the behavior information, pressing of a button for filling a bathtub with hot water, by way of example. The living behavior acquirement apparatus 200 connected to the electrical appliance or the electrical/gas appliance can also function as a control apparatus for controlling the operation of the electrical appliance or the electrical/gas appliance under instructions from the management apparatus 300. For example, upon reception of an instruction to activate the heater from the management apparatus 300, the living behavior acquirement apparatus 200 can output a control signal to activate the heater.

As described above, part of the function of acquiring the behavior information corresponding to the behavior of the user may be provided for the physiological information measurement apparatus 100. In other words, the physiological information measurement apparatus 100 can be configured as the living behavior acquirement apparatus 200, and vice versa (the living behavior acquirement apparatus 200 can be responsible for the function of the physiological information measurement apparatus). Depending on how to acquire the physiological information and the behavior information, the physiological information and the behavior information can be acquired by a single apparatus or by each of independent apparatuses.

The living behavior acquirement apparatus 200 may be formed integrally with the sensor apparatus 201. Specifically, each sensor apparatus 201 can have a communication function to perform data communication with the management apparatus 300, or can have the function of computing and processing detected behavior information into predetermined data or data format. Another example of the living behavior acquirement apparatus 200 is the display apparatus 150 on the user side installed in the user's house. Information acquired through input operation by the user on the display apparatus 150 (such as selection of a "took medicine" button on a medicine-taking check screen) may be collected by the management apparatus 300 as the behavior information.

The management apparatus 300 receives input of the physiological information and the behavior information of the user from the physiological information measurement apparatus 100 and the living behavior acquirement apparatus 200, respectively, estimates the living behavior situation of the user from the input behavior information of the user, and determines the user's health condition based on the estimated living behavior situation and the physiological information input from the physiological information measurement apparatus 100.

The determination of the user's health condition is not limited thereto. The health condition may be estimated based on the physiological information input from the physiological information measurement apparatus 100, the behavior estimation may be performed based on the behavior information input from the living behavior acquirement apparatus 200, and the health condition may be determined from a combination of the behavior estimation result and the health condition estimated based on the physiological information. In other words, the physiological information and behavior information can be used in no particular order in the procedure of determining the health condition.

The management apparatus 300 includes a control unit 310, a storage unit 320, and a communication control unit 330. The control unit 310 includes a behavior estimation section 311 for estimating the living behavior situation of the user based on the behavior information of the user, a health condition determination section 312 for determining the user's health condition based on the estimated living behavior situation and the physiological information of the user, and an output control section 313 for controlling output of watching information to the display apparatus 400 on the watcher side according to the determined health condition.

The communication control unit 330 controls data communication with the physiological information measurement apparatus 100 of the user and the living behavior acquirement apparatus 200 and controls data communication for controlling display of watching screens in the display apparatus 400 on the watcher side and the display apparatus 150 on the user side by the output control section 313.

Each of the display apparatuses 150, 400 is a display apparatus such as a liquid crystal display having a data communication function and is a digital television apparatus, for example. The display apparatuses 150, 400 switch screens through remote control, or display programs or contents selected through remote control following displayed on-screen instructions. Specifically, the user, the family member on the watcher side and the like can perform input (selection) from the display apparatuses 150 and 400 through remote control. The output control section 313 of the management apparatus 300 can receive the selection information input from the display apparatuses 150, 400 and provide screens or contents associated with the selection information for the display apparatuses 150, 400. As described above, the selection information input from the display apparatuses 150, 400 may be transmitted to the management apparatus 300 as the behavior information of the user.

Although the camera (imaging apparatus) and the sound-collecting microphone are installed in the user's house in the above description, they may be attached to the display apparatuses 150, 400. For example when a high-functionality digital television apparatus having a videophone is used, the built-in sound-collecting microphone, camera, and screen touch sensor can be used to acquire the behavior information.

The storage unit 320 stores user's physiological information 321 received from the physiological information measurement apparatus 100 for each user. For example, the storage unit 320 can store times and values of measurement in temporal sequence for each physiological information. The storage unit 320 also stores user's behavior information 322 received from the living behavior acquirement apparatus 200 for each user. For example, the storage unit 320 stores detection information together with times of detection in temporal sequence for each sensor apparatus 201.

Behavior pattern knowledge information 323 is information including previously specified behavior patterns corresponding to detection information detected by the sensor apparatus 201. The behavior estimation section 311 refers to the behavior pattern knowledge information 323 and matches a behavior pattern with corresponding behavior information 322 to identify the living behavior situation of the user.

For example, when detection information from a pressure sensor installed at a place for sleeping such as a mattress or a bed changes from a pressure corresponding to the user's weight to a lower pressure, rising of the user can be known. In this case, an acceleration sensor may determine the user's posture such that the rising of the user can be known based on a plurality of behavior information pieces from the pressure sensor and the acceleration sensor. In the behavior pattern knowledge information 323, for example, the user's behavior of "rising" is previously associated behavior information (such as a pressure change detected by the pressure sensor and a posture detected by the acceleration sensor). The behavior estimation section 311 refers to the behavior pattern knowledge information 323, matches it with behavior information acquired in temporal sequence, and identifies the living behavior situation of the user. The behavior estimation section 311 can arrange and store the estimated living behavior situations in temporal sequence in the storage unit 320 for each user.

A health condition knowledge information 324 is determination information for determining the user's health condition from the physiological information 321. For example, threshold values of blood pressure and body temperature are stored as health condition knowledge. The health condition determination section 312 refers to the health condition knowledge information 324 to determine whether the physiological information 321 is beyond determination values (upper limit and lower limit) in each physiological information or falls within the range from the upper limit to the lower limit specified as the determination values.

For example, when it is determined that none of physiological information pieces are beyond the determination values (all falls between the determination values determined to be normal), the health condition determination section 312 determines that the user's health condition is "normal." When any physiological information piece is beyond the determination values determined to be normal but is not beyond determination values determined to be abnormal, that is, it is not determined to be normal or abnormal, the health condition determination section 312 determines that the user's health condition is "non-normal" as a health condition between "normal" and "abnormal." When it is determined that any one physiological information piece is beyond the determination values (falls between the determination values determined to be abnormal), the health condition determination section 312 determines that the user's health condition is "abnormal."

Each of the determined "normal," "non-normal," and "abnormal" health conditions can be subdivided. For example, the "normal" can be determined in subdivisions of "good" and "so-so." Determination values for each of "good" and "so-so" can be stored as the health condition knowledge information. Similarly, the "abnormal" health condition can be subdivided into "mild" and "severe, "for example. Such arbitrary statuses can be associated with the determination values for determining the health condition, so that the health condition can be determined as an arbitrary status from the physiological information relating to the estimated living behavior situation.

As described above, the health condition knowledge information 324 can store the determination information for determining the health condition as each status such as "normal," "non-normal," and "abnormal," and the determination information can be preset based on a history (statistics) of user's physiological information and information including medical opinions. Alternatively, general determination information can be used regardless of user-specific determination information or each user.

Although the above description has been made in the example including the behavior pattern knowledge information for estimating the living behavior situation and the health condition knowledge information for determining the health condition, additional knowledge information may be included for determining a fault or improper mounting of each device which measures the physiological information or the behavior information. Thus, correct health condition determination can be performed, not only when each device has the function of self-determining a fault and improper mounting, but also when such self-determination fails, by determining a device fault simultaneously with the determination of the health condition.

Health condition information 325 stores the health condition determined by the health condition determination section 312. The health condition information 325 may store the determined health condition in association with the living behavior situation of the user. For example, the living behavior situation of "rising" can be associated with the "normal" health condition determined from physiological information to allow the health condition to be known in association with the living behavior situation in temporal sequence.

Each time the living behavior situation is estimated, the health condition determination section 312 can determine the user's health condition from the physiological information associated with the estimated living behavior situation, and thus can associate determined health conditions with respective living behavior situations. A plurality of determined health conditions in the morning, afternoon, or a day can be averaged, and the averaged health condition may be determined once a day or once in the morning and afternoon.

In the processing of determining the user's health condition by the health condition determination section 312 in the present embodiment, the living behavior situation of the user estimated from the behavior information 322 is taken into account, and the user's health condition is determined from the physiological information 321 and that living behavior situation.

For example, physiological information measured during climbing of stairs is physiological information of the user under an exercise load greater than at rest, so that the user's health condition may not be obtained (determined) accurately. To address this, when a movement of the user is detected in order by a human detection sensor installed at the bottom of the stairs and then a human detection sensor installed at the top of the stairs, the living behavior situation of the user can be determined to be "climbing of stairs." Once the living behavior situation of climbing of stairs is obtained in determining the health condition, a finally determined health condition is "normal" or "non-normal" even when the "abnormal" health condition is formally determined from the determination information.

Similarly, the situation of the user just after a bath can be estimated from detection of entrance to and exit from a bathroom by a human detection sensor or a pressure sensor placed on a bath mat in an undressing room or the like or from behavior information including the use or nonuse of a water heater. In determining the health condition based on physiological information representing the estimated "just after a bath," a finally determined health condition can be "normal" or "non-normal." Such a living behavior situation can be stored previously in the health condition knowledge information 324 as a living behavior situation which is not determined to be "abnormal" even when the "abnormal" health condition is formally determined from the determination information.

Since the health condition determination processing in the present embodiment determines the user's health condition not based on one of the physiological information and the living behavior situation but based on the physiological information associated with the estimated living behavior situation of the user, the health condition can be known accurately. As illustrated in Examples later described, the health condition can be determined by using physiological information in a particular living behavior situation.

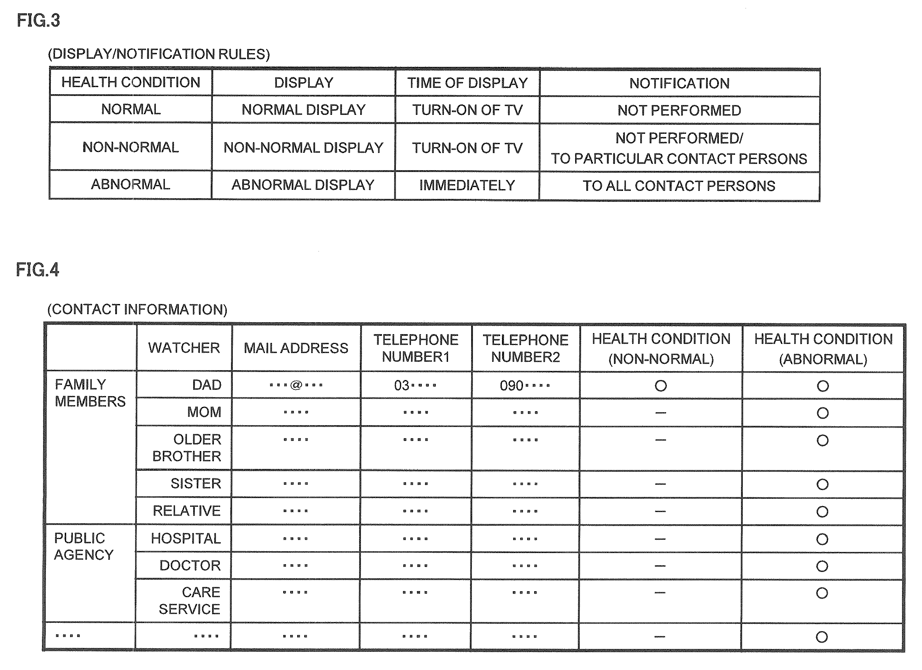

Display/notification rules 326 are information which sets a rule of displaying watching screens and a rule of notifying contact persons on the watcher side as appropriate for the user's health condition determined by the health condition determination section 312. As shown in Fig. 3, when the "normal" health condition is determined, the rules are Set such that a watching screen for normal display 501 is displayed in the display apparatus 400 on the watcher side, and a notification to contact persons on the watcher side is not performed.

When the "non-normal" health condition is determined, a watching screen for non-normal display 502 is displayed in the display apparatus 400 on the watcher side and a notification to contact persons on the watcher side is not performed or is performed to some particular contact persons on the watcher side. When the "abnormal" health condition is determined, a watching screen for abnormal display 503 is displayed in the display apparatus 400 on the watcher side, a notification is performed to all contact persons on the watcher side, and a notification is performed to a public agency (such as an administrative agency, hospital, user's doctor, care service provider) and an security agency other than family members.

Fig. 4 shows an example of contact information 327. As shown in Fig. 4, watcher for watching the user include family members (daddy, mommy, brother, young sister, relative and the like) and public agencies (hospital, user's doctor, care service provider and the like) other than family members, and their contact information (telephone numbers, mail addresses, FAX numbers and the like) is stored. Necessity or non-necessity of notification of each health condition is stored for each watcher. For example, the output control section 313 can extract necessity of notification (O) based on the determined health condition to perform notification processing.

The rules shown in Fig. 3 and Fig. 4 can be arbitrarily set on the user side or system side. However, the display rule and the notification rule when the "abnormal" health condition is determined may be configured not to allow the arbitral setting to be performed on the user side since it is an urgent case.

Screen information 328 stores the watching screens displayed in the display apparatus 400 on the watcher side and health monitor screens displayed in the display apparatus 150 on the user side. The watching screen is a display screen for presenting the determined health condition to the watcher, and a plurality of such watching screens are stored for the respective health conditions. The health monitor screen is a display screen for presenting the determined health condition to the user.

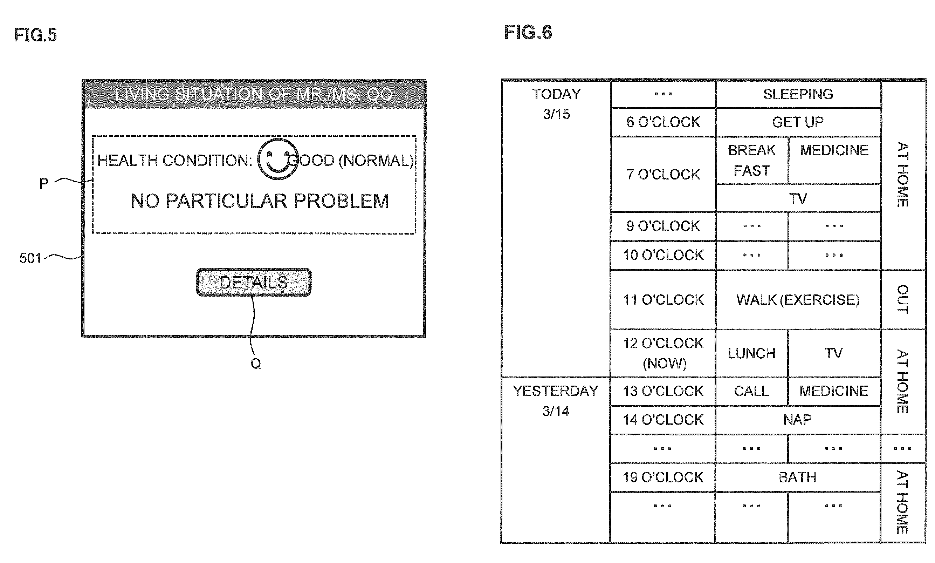

Fig. 5 is a diagram showing an example of the watching screen 501 for the "normal" health condition. As shown in Fig. 5, the watching screen 501 includes a title (living situation of Mr. /Ms. OO), a health condition display field P, and a details button Q. The health condition display field P displays, for example, an icon for "normal (good) " and characters "good (normal)" or "no particular problem."

The details button Q can be formed of a single or a plurality of buttons and is a selection button for displaying various screens showing the user's health condition. For example, Fig. 6 shows an example of a living situation information list screen including estimated living behavior information of the user arranged in temporal sequence. The output control section 313 can use the plurality of living behavior situations estimated in the behavior estimation section 311 to form a list of living behavior situations up to the present time arranged in temporal sequence and display the list in the display apparatus 400 on the watcher side. As described above, the list of the living behavior situations arranged in temporal sequence (living situation information list screen) may be formed by the behavior estimation section 311. In this case, the output control section 313 acquires the list of the living behavior situations arranged in temporal sequence stored in the storage unit 320 and outputs the list to the display apparatus 400 for display.

The output control section 313 can also perform control to display the living behavior situations of yesterday or the past as the living behavior situations of the user after the present time. For example, the living behavior situations of yesterday can be displayed as future living behavior situations after the present time to present a living behavior forecast showing the rhythm of user's life to the watcher side. The living situation information list screen can be produced in display form similar to a television program list. The living situation information list can be controlled to be displayed in parallel to the television program list along the time axis of the television program list.

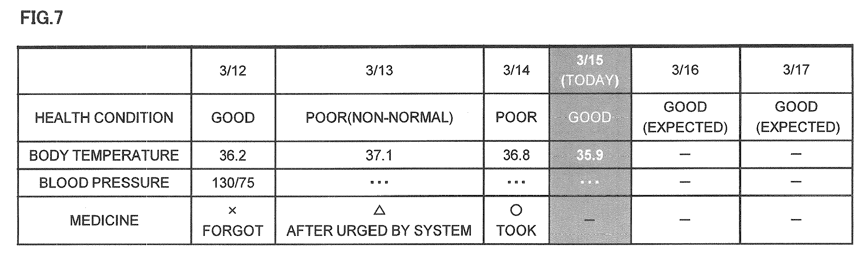

Fig. 7 shows an example of a health situation information screen (wellness forecast) including determined user's health conditions arranged in temporal sequence. As shown in Fig. 7, the output control section 313 can produce the health situation information screen including the results of health condition determination for the past several days arranged in temporal sequence and display the screen in the display apparatus 400 on the watcher side. Future health conditions of tomorrow, day after tomorrow, and afterward can be displayed as the wellness forecast. For example, the tendency to get better physical condition due to taking medicine can be previously observed, and this expected effect triggered by taking medicine can be displayed as a health condition forecast on the wellness forecast screen. In this case, the health condition determination section 312 refers to the health condition knowledge information 324 to determine the future (tomorrow, day after tomorrow) health condition of the user by factoring in the possible better physical condition due to taking medicine.

In the example of Fig. 7, the health situation information screen can include a medicine taking situation. For example, the health situation information screen can be configured such that the user's health condition for each day includes information representing that the user forgot to take medicine (X), that the user took medicine after urged (attention called) by the life watching and support system (Δ), or that the user took medicine without being urged by the life watching and support system (O).

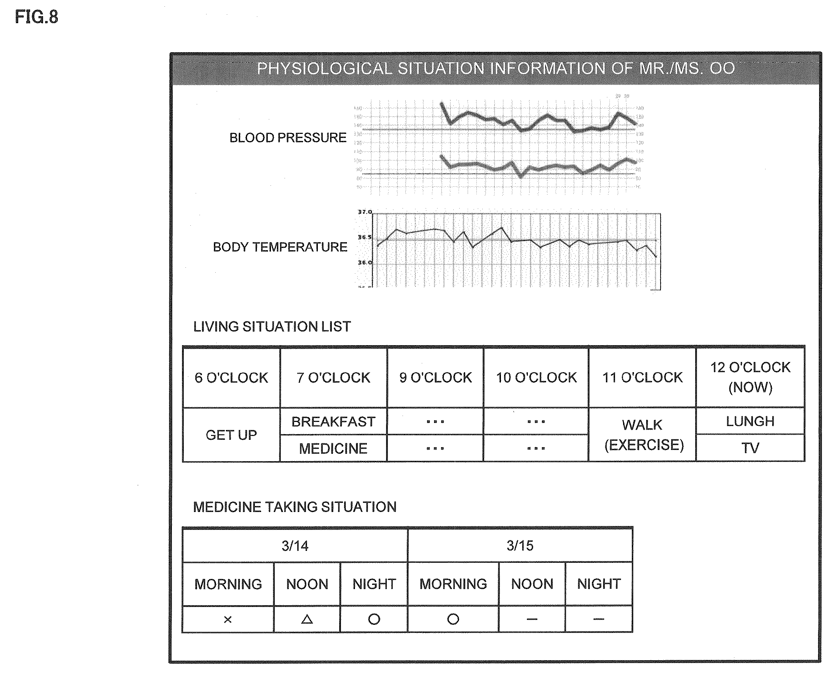

Fig. 8 shows an example of a physiological situation information screen showing, in graphical form, user's physiological information in temporal sequence. As shown in Fig. 8, the output control section 313 can produce the physiological situation information screen representing in temporal sequence a single or a plurality of physiological information pieces acquired up to the present time and display the screen in the display apparatus 400 on the watcher side. The output control section 313 can produce the physiological situation information screen additionally displaying the living situation information including the estimated living behavior information of the user arranged in temporal sequence shown in Fig. 6 along the time axis for the physiological information and additionally displaying the medicine taking situation shown in Fig. 7.

The screens shown from Fig. 6 to Fig. 8 may be displayed by default in the watching screen 501 without selection of the details button.

The watching screen 502 for the "non-normal" health condition is basically the same as the screen for the "normal" health condition except that the screen 502 displays an icon for the "non-normal" health condition and characters "poor physical condition" or "seem to have cold." Unlike the watching screen 501, the watching screen 502 can display the health situation information shown in Fig. 7 or the physiological situation information shown in Fig. 8 by default in view of the poor health condition.

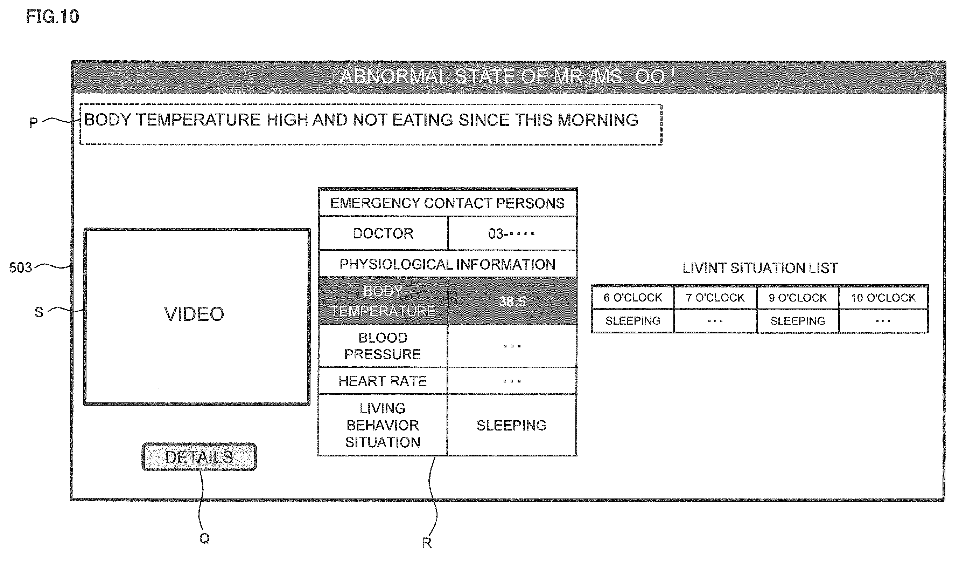

Fig. 10 is a diagram showing an example of a watching screen 503 for the "abnormal" health condition. As shown in Fig. 10, the watching screen 503 for the "abnormal" health condition is a screen including information based on the urgency of the "abnormal" health condition, unlike the screens for the "normal" and "non-normal" health conditions.

As shown in Fig. 10, the watching screen 503 for the "abnormal" health condition is controlled to display a message indicating "abnormality" on a health condition display field P and to output an audio message "health condition of Mr./Ms. OO is abnormal" from a speaker of the display apparatus 400. The screen 503 also displays an emergency information display field R including emergency contact persons, physiological information, and living behavior situation, and a video display field S displaying an image or a video of the user or the interior of the house taken by an imaging apparatus installed in the user's house. The watching screen 503 can also display the living situation information list screen shown in Fig. 6. Although the display example in Fig. 10 shows the situation on the day when the abnormality occurs, the living situation before that day can be displayed by operating the display apparatus 400 on the watcher side.

The output control section 313 acquires the emergency contact persons, physiological information, and latest living behavior situation from the contact information 327, physiological information 321, and behavior information 322, respectively, and displays them in the emergency information display field R. The output control section 313 can emphasize, for example by highlighting, the physiological information of a plurality of physiological information pieces that is relied on in the determination of the "abnormal" health condition. The living behavior situation associated with the physiological information determined to be abnormal or the latest living behavior situation is displayed.

The output control section 313 controls the display of the health monitor screen in the display apparatus 150 on the user side. The health monitor screen is a display screen for presenting the determined daily health condition to the user.

Basically, the health monitor screen can consist of a plurality of health monitor screens for the respective statuses including the "normal," "non-normal," and "abnormal" health conditions, similarly to the watching screen. Each health monitor screen has the same screen configuration (screen layout) and the same displayed information as the watching screen.

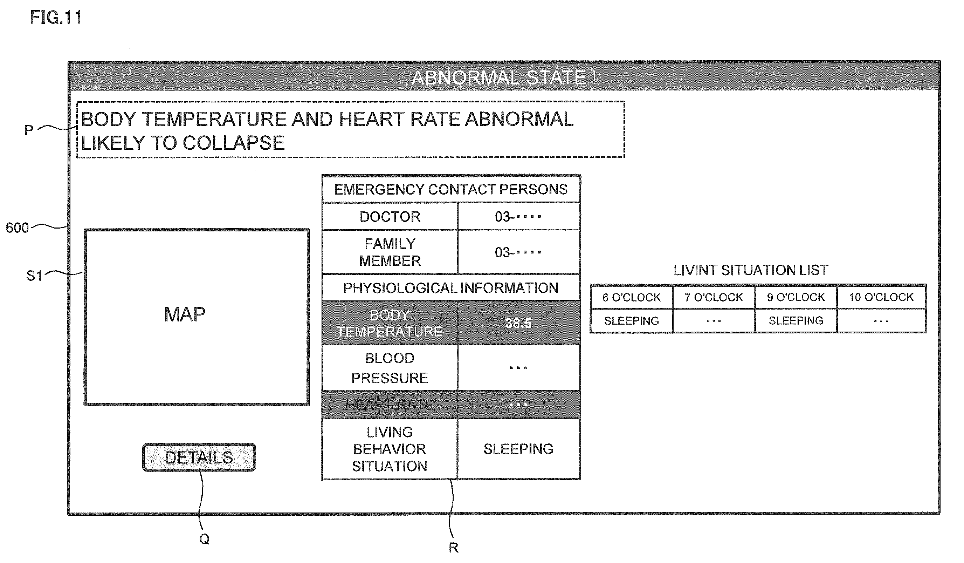

Fig. 11 shows an example of a health monitor screen 600 (corresponding to second health monitor screen) when the "abnormal" health condition is determined. As shown in Fig. 11, the health monitor screen 600 has a configuration similar to the watching screen 503, but a health condition display field P outputs a message for the "abnormal" health condition such as "abnormal body temperature and heart rate," "likely to collapse." A map display field S1 is displayed instead of the video display field, and an emergency information display field S shows the contact information of a family member or a relative as an emergency contact person.

The map display field S1 can display, on a map, the nearest hospital or the house of the user's doctor or the family member included in contact information 327, or can display the location where life-saving equipment such as an AED is placed.

The health monitor screen 600 shown in Fig. 11 when the "abnormal" health condition is determined is displayed to include useful information for a person who helps the user. The health monitor screen 600 is configured to include information for assisting such a person in giving immediate aid to the user by referring to the map information to find a route to the nearest hospital or seeing the emergency contact persons.

In displaying the health monitor screen when the "abnormal" health condition is determined, a screen for requiring the user to check may be displayed. For example, when the "abnormal" health condition is determined due to a fault of the physiological information measurement apparatus 100, the user may have no abnormality in the health condition. In this case, an erroneous determination or an erroneous notification can be prevented by displaying the screen for prompting the user to input. The check screen showing a message such as "OK?" or "want to contact family member?" is displayed in the display apparatus 150 on the user side for a predetermined time period. Then, when no response (input) is made, the display is switched to the health monitor screen 600 shown in Fig. 11. When any response is made, the health monitor screen 600 shown in Fig. 11 is not displayed and a notification to the watcher is not performed.

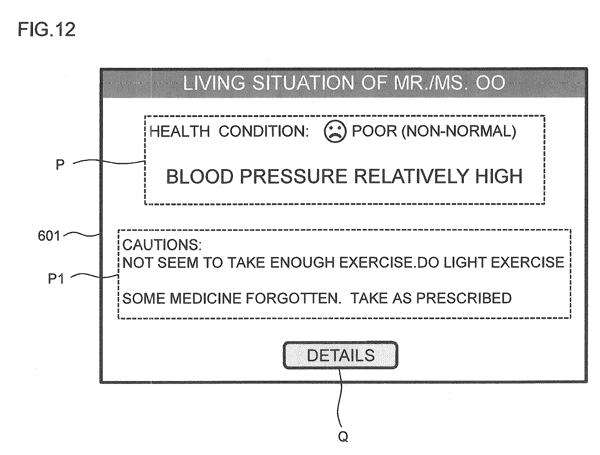

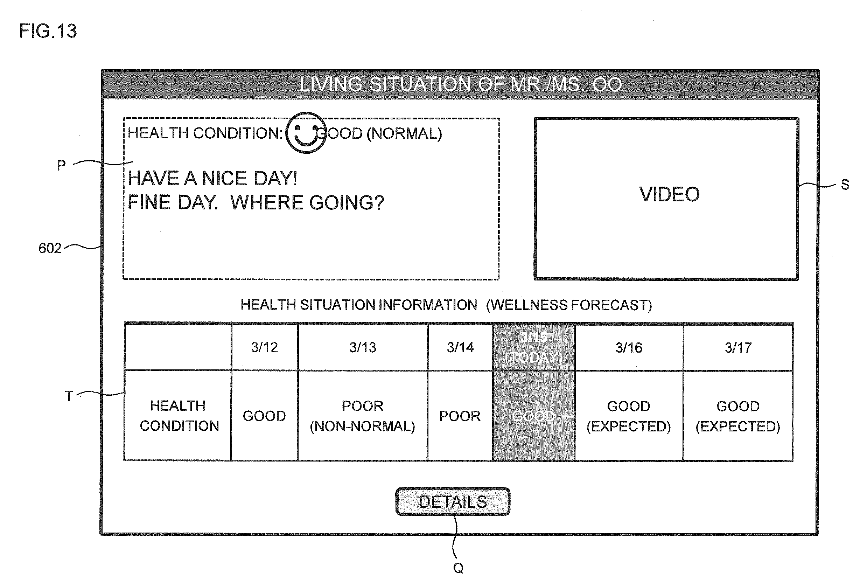

Other examples of the health monitor screen displayed in the display apparatus 150 on the user side include health monitor screens 601, 602 shown in Fig. 12, Fig. 13, respectively. Fig. 1L shows the health monitor screen 601 (corresponding to a third health monitor screen) when the user's health condition is "non-normal." A health condition display field P can display a message such as "blood pressure relatively high" and display a message such as a comment or cautions to the user side.

The example of Fig. 12 is configured to display a message about prevention. For example, it can be seen from the living behavior situation that exercise is reduced or confirmation of medicine taking is not input (medicine is not taken). The health condition determination section 312 can perform prevention determination processing of matching determination information about prevention stored in the health condition knowledge information 324 with the living behavior situation, independently of or together with the health condition determination processing. The output control section 313 can display a prevention message pointing out a lack of living behaviors that may cause poorer physical condition in order to promote prevention for the user, although the "abnormal" health condition is not determined.

Fig. 13 shows the health monitor screen 602 (corresponding to a first health monitor screen) when the user health condition is "normal." A health condition display field P can display a message such as "have a nice day" and display a message such as a comment about good physical condition to the user side. The example of Fig. 13 includes a display field T displaying the health situation information shown in Fig. 7 and video (image) display field S. The video (image) display field S can display a video or an image transmitted from a family member such as a grandchild together with a message.

Next, the processing performed by the output control section 313 is described. As described above, the output control section 313 of the present embodiment performs the display control for displaying the watching screen in the display apparatus 400 on the watcher side.

For example, as shown in Fig. 3, the output control section 313 can display the watching screen at the time when the watcher switches on the display apparatus 400. When the health condition determined by the health condition determination section 312 is "normal" or "non-normal," and the output control section 313 receives information of the switch-on of the display apparatus 400 over the network, the output control section 313 acquires the health condition and the watching screen 501 from the storage unit 320, and outputs and displays the watching screen 501 including the health condition (normal or non-normal) in the display apparatus 400.

Other than the time when the watcher switches on the display apparatus 400, for example when a predetermined button is pressed on a remote controller during viewing of a television program in the display apparatus 400, the output control section 313 can switch from the currently viewed screen to output and display the watching screen 501 including the health condition (normal or non-normal) in the display apparatus 400. In this case, the currently viewed screen and the watching screen 501 may be displayed together in dual-screen form (by switching to dual-screen display including the watching screen 501). An icon representing the determined health condition may be displayed at a corner of the currently viewed screen.

The output control section 313 can display the watching screen in the display apparatus 400 at the time when the watcher switches off the display apparatus 400. When the health condition determined by the health condition determination section 312 is "normal" or "non-normal," the output control section 313 can receive information of the switch-off of the display apparatus 400 over the network, output and display the watching screen 501 including the health condition (normal or non-normal) in the display apparatus 400 for several seconds, and then stop the display.

When the health condition determined by the health condition determination section 312 is "abnormal," the output control section 313 forcedly displays the watching screen 503 for abnormal display in the display apparatus 400 regardless of an active action performed by the watcher side on the display apparatus 400.

At the time when the "abnormal" health condition is determined by the health condition determination section 312, the output control section 313 acquires the watching screen 503, contact information 327, and living behavior situation from the storage unit 320, and outputs and displays the watching screen 503 in the display apparatus 400. When the switch of the display apparatus 400 is off, the output control section 313 outputs a control signal for switch-on to the display apparatus 400 and controls the display apparatus 400 to forcedly display the watching screen 503.

While the display apparatus 400 is displaying a television program or the watching screen 501 or the like, the output control section 313 performs control to forcedly switch from the currently viewed television screen or the watching screen 501 to the watching screen 503.

Since the output control section 313 of the present embodiment displays the watching screen including the determined health condition in the display apparatus 400 on the watcher side depending on the health condition in this manner, the watcher side can know the daily health condition of the user.

Since the output control section 313 selectively displays, in the display apparatus 400, the watching screen 501 for "normal" determination (corresponding to the first watching screen) when the "normal" health condition is determined or the watching screen 503 for emergency (corresponding to the second watching screen) for notifying that an abnormal state occurs in the user when the "abnormal" health condition is determined, the watcher side can quickly and clearly know the abnormality occurring in the user's health condition through the display apparatus 400.

When the "normal" health condition is determined, the watching screen 501 is displayed in the display apparatus 400 at an arbitrary time based on an active operation input to the display apparatus 400 on the watcher side. When the "abnormal" health condition is determined, the watching screen 503 is forcedly displayed in the display apparatus 400 regardless of an active operation input to the display apparatus 400 on the watcher side. Thus, when an abnormality occurs, the watcher side can be notified quickly, and when no abnormality occurs (normal or non-normal), the user's health condition can be known visually in the same manner as viewing of a television program, so that the watcher side is not forced to watch the user, that is, watching the user at all times is not required. This can achieve an environment for watching the user with a reduced burden on the watcher side.

The output control section 313 also performs display control for displaying the health monitor information screens 600, 601, and 602 in the display apparatus 150 on the user side. Since the display control can be performed similarly to the control of the watching screen including display timing control, detailed description is omitted.

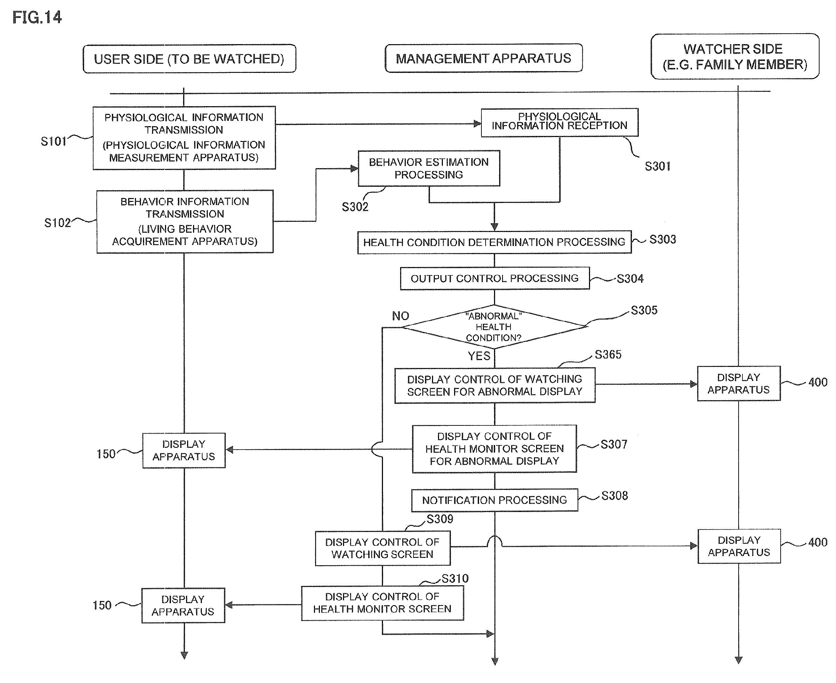

Fig. 14 is a flow chart showing a flow of processing in the life watching and support system. The following description is made assuming that a user wears the physiological information measurement apparatus 100.

The physiological information measurement apparatus 100 starts measurement of physiological information and transmits the measured physiological information to the management apparatus 300 (S101). The management apparatus 300 receives the physiological information in temporal sequence and stores the information in the storage unit 320 (S301). Similarly, the living behavior acquirement apparatus 200 transmits detection information output from the sensor apparatus 201 to the management apparatus 300 as behavior information (S102). The management apparatus 300 stores the behavior information received in temporal sequence in the storage unit 320.

The management apparatus 300 estimates a living behavior situation of the user by using the received behavior information. The behavior estimation section 311 refers to the behavior pattern knowledge information 323 based on the received behavior information and matches a behavior pattern with the corresponding behavior information to identify the living behavior situation of the user (S302). The estimated living behavior situation is stored in the storage unit 320.

The management apparatus 300 determines a health condition of the user by using the received physiological information and the estimated living behavior situation. The health condition determination section 312 refers to the health condition knowledge information 324 based on physiological information associated with the estimated living behavior situation of the user to determine whether the user's health condition is "normal," "non-normal," or "abnormal" (S303). The determined health condition is stored in the storage unit 320 (health condition information 325).

The management apparatus 300 displays a watching screen including the determined health condition in the display apparatus 400 on the watcher side depending on the health condition (S304). When the "normal" or "non-normal" health condition is determined (NO at S305), the output control section 313 displays the watching screen 501 or the watching screen 502 in the display apparatus 400 at an arbitrary time based on an active operation input to the display apparatus 400 on the watcher side (5309) . The output control section 313 displays a health monitor screen in the display apparatus 150 at an arbitrary time based on an active operation input to the display apparatus 150 on the user side (S310).

When the "abnormal" health condition is determined (YES at S305), the output control section 313 forcedly displays the watching screen 503 for abnormal display in the display apparatus 400 regardless of an active operation input to the display apparatus 400 on the watcher side (S306). The output control section 313 forcedly displays the health monitor screen 601 shown in Fig. 11 when the "abnormal" health condition is determined in the display apparatus 150 (S307). The output control section 313 notifies the cellular phone, fixed-line telephone, or computer of the watcher that an abnormality occurs in the user based on the display/notification rules 326 (S308).

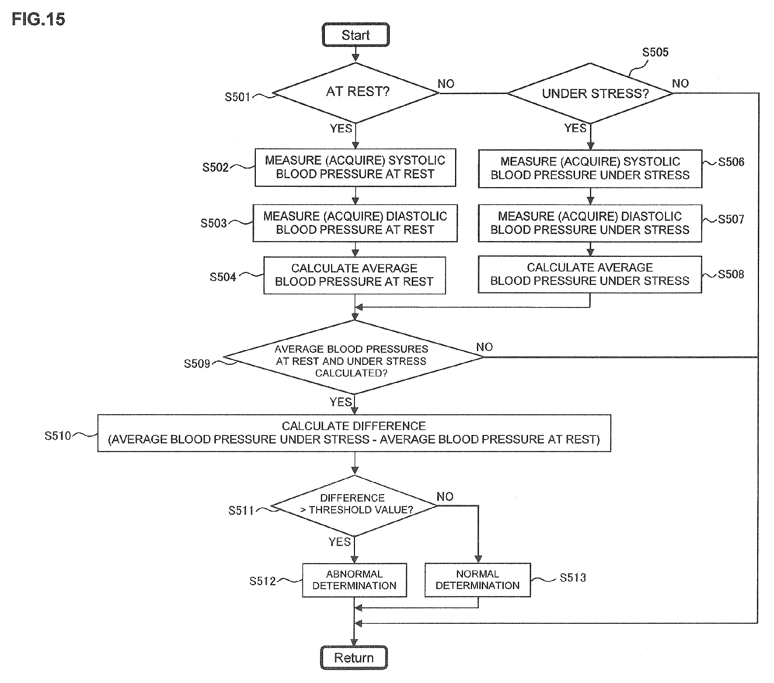

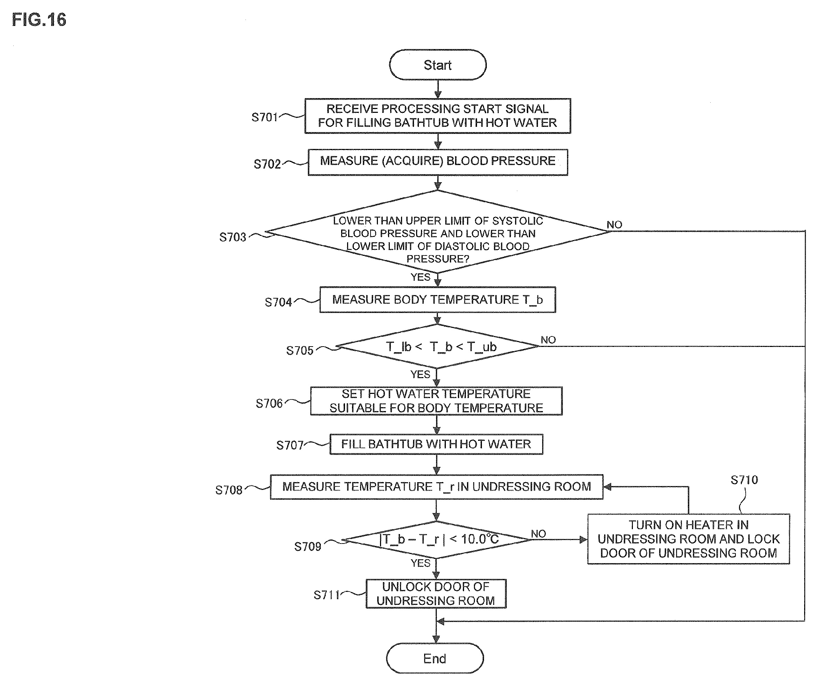

Fig. 15 and Fig. 16 show exemplary uses of the life watching and support system (Examples).

### EXAMPLE 1

Fig. 15 shows an example of health condition determination processing of determining a health condition of a user through comparison between blood pressure values under exercise stress and blood pressure values at rest.

First, the behavior estimation section 311 estimates a living behavior situation of the user from behavior information of the user acquired by the living behavior acquirement apparatus 200 and determines whether or not the estimated living behavior situation is "at rest" (S501). For example, when a pressure is detected by a pressure sensor placed on a mattress, it can be estimated that the user is at rest sleeping on the mattress. Alternatively, it can be estimated that the user is at rest having a chat in a living room by a human detection sensor on the ceiling or wall of the living room, a sound sensor in the living room, the presence or absence of operation of a television apparatus, and a pressure sensor placed on a cushion in the living room.

When the living behavior situation of the user is determined to be at rest (YES at S501), the health condition determination section 312 measures blood pressure values in the estimated living behavior situation (systolic blood pressure, diastolic blood pressure), that is, blood pressure values (systolic blood pressure, diastolic blood pressure) at the time when the living behavior situation is estimated (S502, S503). From the measured blood pressure values, an average blood pressure at rest ((systolic blood pressure - diastolic blood pressure)/3 + diastolic blood pressure) is calculated (S504).

The behavior estimation section 311 estimates the living behavior situation of the user from the behavior information of the user acquired by the living behavior acquirement apparatus 200 and determines whether or not the estimated living behavior situation is "under exercise stress" (S505). For example, when a movement of the user is detected in order by a human detection sensor placed at the bottom of stairs and a human detection sensor placed at the top of the stairs, the living behavior situation of the user can be estimated to be under exercise stress of "climbing of stairs."

When the living behavior situation of the user is determined to be under exercise stress (NO at S501 and YES at S505), the health condition determination section 312 measures blood pressure values (systolic blood pressure, diastolic blood pressure) at the time when the living behavior situation is estimated (S506, S507). From the measured blood pressure values, an average blood pressure under exercise stress ((systolic blood pressure - diastolic blood pressure)/3 + diastolic blood pressure) is calculated (S508).

Once the average blood pressures are calculated at steps S504 and S508 (YES at S509), the health condition determination section 312 subtracts the average blood pressure at rest from the average blood pressure under exercise stress to calculate a difference (S510). It is determined whether or not the difference is larger than a predetermined threshold value (S511). When the difference is larger than the threshold value, the health condition determination section 312 determines that the user's health condition is "abnormal" (S512). When the difference is smaller than the threshold value, the health condition determination section 312 determines that the user's health condition is "normal" (S513).

The difference calculated by subtracting the average blood pressure at rest from the average blood pressure under exercise stress can be displayed in graphical form on the watching screen 500 similarly to daily blood pressure values as shown in Fig. 8. The average blood pressures may be calculated in any other method.

The determination of the health condition using the average blood pressure under exercise stress and the average blood pressure at rest is based on the fact that an excessive increase in blood pressure under exercise stress is found in development of arteriosclerosis due to metabolic syndrome (for reference, http://medical.nikkeibp.co.jp/leaf/all/gakkai/jsh2010/201010/51704 3.html). In the life watching and support system, the living behavior situation estimated form the behavior information of the user is accurately obtained, and the user's health condition is obtained from physiological information associated with the obtained living behavior situation, the user's health condition can be appropriately known from physiological information acquired in combination with various living behaviors of the user, instead of determined from physiological information measured at each time. In addition, the determination of the health condition using the physiological information under exercise stress and at rest can provide the health condition including prevention information for the watcher and the user.

When the blood pressure value at rest (systolic blood pressure or diastolic blood pressure) exceeds a predetermined threshold value or when the blood pressure value under exercise stress exceeds a predetermined threshold value, the health condition determination section 312 can determine the user's health condition to be "abnormal. " The user's health condition may be determined by using the average blood pressures at rest and under exercise stress.

### EXAMPLE 2

The present example is an aspect in which the life watching and support system is applied to prevention of a heat shock phenomenon. The heat shock refers to the effect of a sudden temperature change on a body. When a person moves between places at greatly different temperatures such as between a living room or a bathroom and an undressing room or a toilet, the body is exposed to the different temperatures and the blood pressure is quickly changed, which may lead to a stroke or a myocardial infarction.

In the present example, a living behavior situation of a user estimated from the viewpoint of watching the user is used to determine whether or not the user is in an environment in which a risk is likely to arise in the health condition, and when the user is in such a risky environment, the behavior of the user is supported to avoid (prevent) a health risk by the management apparatus 300.

As shown in Fig. 16, when a button for filling a bathtub with hot water is pressed, the living behavior acquirement apparatus 200 transmits a signal indicating the hot-water filling button pressing to the management apparatus 300. The management apparatus 300 starts health risk prevention processing in response to the reception of the signal indicating the hot-water filling button pressing (S701). Specifically, the behavior estimation section 311 of the management apparatus 300 estimates a living behavior situation in which the user takes a bath from the signal indicating the hot-water filling button pressing and starts the health risk prevention processing. The management apparatus 300 controls the physiological information measurement apparatus 100 to measure the blood pressure of the user and acquires the blood pressure of the user in association with the living behavior of the user pressing the hot water filling button from the physiological information measurement apparatus 100 (S702).

The health condition determination section 312 determines whether or not the blood pressure value of the user (systolic blood pressure and/or diastolic blood pressure) falls within a predetermined threshold range. For example, it is determined whether the systolic blood pressure is smaller than a predetermined upper limit and whether the diastolic blood pressure is larger than a predetermined lower limit (S703). In the determination processing at step S703, other physiological information can be added to the determination conditions. For example, whether or not an abnormal brain wave pattern is found in brain waves and whether or not an abnormal behavior pattern is found in behavior information can be added to the determination conditions.

When it is determined that the blood pressure value of the user falls within the predetermined threshold range, the health condition determination section 312 outputs a signal for measuring a body temperature T_b of the user to the physiological information measurement apparatus 100 to acquire the body temperature T_b of the user (S704). The health condition determination section 312 determines whether or not the measured body temperature T_b lies between a preset lower limit of normal body temperature T_lb and a preset upper limit of normal body temperature T_ub (S705). When the user's body temperature T_b lies between the lower limit of normal body temperature T_lb and the upper limit of normal body temperature T_ub, a hot water temperature is set suitably for the body temperature T_b (S706) and a control signal is output for starting hot water filling into a bathtub at the set hot water temperature through the living behavior acquirement apparatus 200 (S707). The hot water temperature can be set, for example, at a temperature calculated by adding a predetermined value to the body temperature T_b.

Next, the health condition determination section 312 measure a temperature T_r in the undressing room after the start of hot water filling. The health condition determination section 312 instructs the living behavior acquirement apparatus 200 to acquire a temperature detected by a temperature sensor installed in the undressing room and acquires the temperature T_r in the undressing room through the living behavior acquirement apparatus 200 (S708).

When the temperature difference between the body temperature T_b and the temperature T_r in the undressing room is larger than a predetermined value (for example, 10°C) (S709), the health condition determination section 312 performs control such that a heater installed in the undressing room is turned on to heat the undressing room (S710) . The health condition determination section 312 outputs a control signal for activating the heater through the living behavior acquirement apparatus 200 and performs control to heat the undressing room until the temperature difference between the body temperature T_b and the temperature T_r in the undressing becomes smaller than the predetermined value. When a door of the undressing room can be automatically locked, the door is locked.

When the temperature difference between the body temperature T_b and the temperature T_r in the undressing room becomes smaller than the predetermined value by operating the heater installed in the undressing room, the health condition determination section 312 performs control to turn off the heater and unlocks the door of the undressing room (S711). The health condition determination section 312 may perform control to manage the temperature in the undressing room and unlock the door in time for the end of the hot water filling.

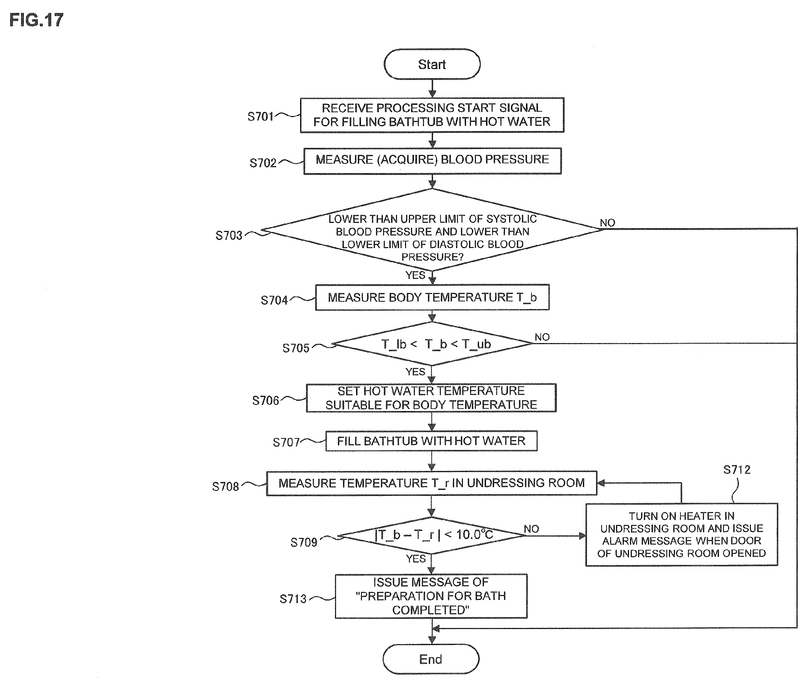

Fig. 17 shows a modification of the health risk prevention processing shown in Fig. 16. An example of Fig. 17 differs from that of Fig. 16 in steps corresponding to steps S710, S711 in Fig. 16. In the example of Fig. 17, when the door of the undressing room is opened during the control for heating the undressing room by turning on the heater installed in the undressing room at step S712, an audio announcement which requests closing of the door of the undressing room is output from a speaker installed in the undressing room through the living behavior acquirement apparatus 200. At step S713, when the temperature difference between the body temperature T_b and the temperature T_r in the undressing room becomes smaller than the predetermined value, a message indicating that preparation for a bath is completed can be issued to the user with audio or output on the display apparatus. In this case, the message indicating that preparation for a bath is completed may be output to the user simultaneously with completion of hot water filling.

As described above, the present example can support the behavior of the user to prevent the health risk based on the physiological information of the user and the environmental information (such as room temperature) by the management apparatus 300, and the estimated living behavior situation of the user can be used to protect the user against an environment in which a risk is likely to arise in the health condition.

The results of the health risk prevention processing in the present example may be provided for the watcher side by the management apparatus 300. For example, the watcher such as a family member can be notified by watching information on the watching screen 500 that the health risk prevention processing was performed before the user took a bath to protect (prevent) the user against the heat shock phenomenon. Such a configuration allows the watcher to know that the user behavior was supported to prevent the health risk, thereby achieving a watching environment with a reduced burden on the watcher.

In the following, other aspects are described than Example 1 and Example 2.

Examples of the trigger for sensing mounting of the physiological information measurement apparatus 100 to start automatic measurement include a difference limited within a predetermined range and a correlation found between the temperature detected by the physiological information measurement apparatus 100 and the room temperature acquired as the behavior information, a sudden change in detected temperature due to measurement of the body temperature in response to mounting of the physiological information measurement apparatus 100, or continuous detection of temperature of approximately 36°C.

Next, an example of the health condition determination processing when the user's body temperature changes is described. When the body temperature measured by the physiological information measurement apparatus 100 is a predetermined value or higher, the health condition determination section 312 performs processing as "alarm" at this point. Then, a living behavior situation is estimated from behavior information acquired by the living behavior acquirement apparatus 200. For example, when it is determined that the living behavior situation is "after change of clothes" in the undressing room (just after bath), the health condition determination section 312 determines that the cause of the body temperature rise is "just after bath" and temporarily determines that the health condition is "normal." The health condition determination section 312 continues to acquire physiological information after that, and confirms the determination that the health condition is "normal" after the body temperature gradually reduces to normal body temperature.

Thus, the health condition determination section 312 performs the alarm determination for the body temperature rise obtained from the physiological information and then determines the cause of the body temperature rise (living behavior situation) with the processing of estimating the behavior of the user. When the cause of the body temperature rise is a living behavior involving such a body temperature rise, the alarm determination is replaced with normal determination based on the subsequent measurement of physiological information to confirm the "normal" health condition of the user. When the cause of the body temperature rise is not a living behavior involving such a body temperature rise at the time of the behavior estimation, the alarm determination is maintained to confirm the user health condition as "abnormal."

Next, an example of the health condition determination processing based on user's brain waves is described. Since brain waves are not stable at first after the physiological information measurement apparatus 100 is mounted, the health condition determination section 312 performs tentative determination as "alarm" at this point. The behavior estimation section 311 estimates a living behavior situation of the user from behavior information detected by an acceleration sensor or a vibration sensor and estimates that the user is changing clothes, for example. The health condition determination section 312 determines that the cause of the unstable brain waves is the behavior of "change of clothes" (determines that the data is unstable since the contact between the physiological information measurement apparatus 100 and the user body is not stable) and temporarily determines that the health condition is "normal." The physiological information representing the unstable brain waves during change of clothes can be stored in the storage unit 320 as determination information for determining the health condition. The health condition determination section 312 continues to acquire physiological information after that, and confirms the determination that the health condition is "normal" after the brain waves stabilize to normal values.

Next, an example of "abnormal" determination in the health condition determination processing for the user is described. When the heart rate measured by the physiological information measurement apparatus 100 exceeds a threshold value, the health condition is not immediately determined to be "abnormal" but determination is made by factoring in the possibility that an abnormality (removal) of the physiological information measurement apparatus 100 may be the cause. Specifically, the health condition determination section 312 checks whether the behavior of the user suddenly collapsing is estimated from behavior information detected by an acceleration sensor or the sound of the user collapsing can be collected by the sound-collecting microphone in the behavior information. When the user does not suddenly collapse or the sound of the user collapsing is not collected (no sound), the health condition determination section 312 assumes that the abnormal heart rate is due to a fault of the physiological information measurement apparatus 100 and determines that the health condition is "normal." In contrast, when the user suddenly collapses or the sound of the user collapsing can be collected, the health condition determination section 312 assumes that the abnormal heart rate is caused not by a fault of the physiological information measurement apparatus 100 but by an abnormality in body or mind of the user and determines that the health condition is "abnormal."

The notification rule when the "abnormal" health condition is determined can be set such that, when the situations (such as going out) of family members of contact persons can be known, the priorities of the contact persons are preset and a notification is first performed with a mail or a voice message (telephone) to the person of the highest priority, and if no response is made from the person of the highest priority, the person of the second highest priority can be notified.

The mental condition of the user can be obtained from the living behavior situation. Even when no particular abnormality is found in physiological information measured by the physiological information measurement apparatus 100, an estimated living behavior situation can be used to estimate, for example, that the user is reclusive. For example, the behavior estimation section 311 can obtain living behavior situations of the user such as not going out of home (door sensor), not watching television (power ON/OFF), not watching a frequently watched program, leaving a particular TV channel on, no laughter included in the user's voice collected by the sound-collecting microphone, and not answering a ringing telephone, and the health condition determination section 312 can determine poor mental health from those living behavior situations.

To reduce the health risk while the user goes out, the user's health condition can be determined and the user or the watcher can be notified that the health risk is increased. For example, the behavior estimation section 311 can estimate that the user is "preparing for going out" from behavior information including the sound of change of clothes, power-off of a television, and turn-off of a light. The health condition determination section 312 determines abnormal or normal physiological information and refers to a history of medicine taking. For example, when no latest history of medicine taking is present, it is determined that the risk of a high blood pressure while the user goes out is increased, and the output control section 313 outputs a medicine-taking check message or an audio guidance with the display apparatus 150. In response, the user can input the completion or incompletion of medicine taking to the display apparatus 150.

Each of the functions of the life watching and support system (management apparatus 300) of the present embodiment can be configured as a program. For example, a program for each function of the life watching and support system can be stored on an auxiliary storage apparatus, not shown, of a computer, a control unit such as a CPU can read the program stored on the auxiliary storage apparatus to a main storage apparatus, and the program read to the main storage apparatus can be executed by the control unit to cause the computer to perform the function of each component in the present embodiment. Thus, the computer on which the program for each function of the life watching and support system of the present embodiment is installed can operate as a computer apparatus which achieves each function of the life watching and support system of the present embodiment.

The program may be recorded on a computer readable recording medium and provided for a computer. Examples of the computer readable recording medium include optical disks such as a CD-ROM, phase-change optical disks such as a DVD-ROM, magneto-optical disks such as a Magnet-Optical (MO) disk and Mini Disk (MD), magnetic disks such as a floppy disk® and removable hard disk, and memory cards such as a compact flash®, smart media, SD memory card, and memory stick. Hardware apparatuses such as an integrated circuit (such as an IC chip) designed and configured specifically for the purpose of the present invention are included in the recording medium.

Although some embodiments of the present invention have been described, those embodiments are illustrative and are not intended to limit the scope of the present invention. The novel embodiments can be implemented in various other forms, and various omissions, substitutions, and modifications can be made thereto without departing from the spirit or scope of the present invention. These embodiments and their variations are encompassed within the spirit or scope of the present invention and within the invention set forth in the claims and the equivalents thereof.

### DESCRIPTION OF THE REFERENCE NUMERALS

- 100: PHYSIOLOGICAL INFORMATION MEASUREMENT APPARATUS
- 101: COMMUNICATION SECTION
- 102: SENSOR MODULE
- 150: DISPLAY APPARATUS
- 200: LIVING BEHAVIOR ACQUIREMENT APPARATUS
- 201: SENSOR APPARATUS
- 202: PROCESSING SECTION
- 203: COMMUNICATION SECTION
- 300: MANAGEMENT APPARATUS (LIFE WATCHING AND SUPPORT APPARATUS)
- 310: CONTROL APPARATUS
- 311: BEHAVIOR ESTIMATION SECTION
- 312: HEALTH CONDITION DETERMINATION SECTION
- 313: OUTPUT CONTROL SECTION
- 320: STORAGE UNIT
- 321: PHYSIOLOGICAL INFORMATION
- 322: BEHAVIOR INFORMATION
- 323: BEHAVIOR PATTERN KNOWLEDGE INFORMATION
- 324: HEALTH CONDIDTION KNOWLEDGE INFORMATION
- 325: HEALTH CONDIDTION INFORMATION
- 326: DISPLAY/NOTIFICATION RULES
- 327: CONTACT INFORMATION
- 328: SCREEN INFORMATION
- 330: COMMUNICATION CONTROL UNIT
- 400: DISPLAY APPARATUS
- 500: WATCHING SCREEN
- 600: HEALTH MONITOR SCREEN

## Claims

1. A life watching and support apparatus connected to a living behavior acquirement apparatus configured to acquire behavior information of a user, connected to a physiological information acquirement apparatus configured to acquire physiological information of the user, and connected to a display apparatus on a watcher side watching the user, the apparatus comprising:
a behavior estimation section configured to estimate a living behavior situation of the user based on the behavior information of the user;
a health condition determination section configured to determine a health condition of the user based on the estimated living behavior situation and the physiological information; and
an output control section configured to display a watching screen including the determined health condition in the display apparatus on the watcher side depending on the health condition.

2. The life watching and support apparatus according to claim 1, wherein the health condition determination section uses preset determination information for each of normal and abnormal determinations to determine the health condition based on the estimated living behavior situation and the physiological information,
the output control section selectively display, in the display apparatus, a first watching screen for normal determination when the health condition is determined to be normal and a second watching screen for emergency for notifying that an abnormal state occurs in the user when the health condition is determined to be abnormal,
when the health condition is determined to be normal, the output control section displays the first watching screen in the display apparatus at an arbitrary time based on an operation input to the display apparatus on the watcher side, and
when the health condition is determined to be abnormal, the output control section displays the second watching screen in the display apparatus regardless of an operation input to the display apparatus on the watcher side.

3. The life watching and support apparatus according to claim 2, wherein the health condition determination section determines that the health condition determined to be not abnormal or normal is non-normal in determination of the health condition based on the estimated living behavior situation and the physiological information with the preset determination information for each of normal and abnormal determinations, and
the output control section displays a third watching screen for non-normal determination when the health condition is determined to be non-normal at an arbitrary time based on an operation input to the display apparatus on the watcher side instead of the first watching screen.

4. The life watching and support apparatus according to claim 2 or 3, wherein the output control section displays, on the second watching screen, the physiological information associated with the health condition determined to be abnormal, the estimated living behavior situation associated with the health condition determined to be abnormal, or a video acquired by an imaging apparatus installed in a house of the user.

5. The life watching and support apparatus according to claim 3, wherein the life watching and support apparatus is connected to a display apparatus on a user side, and
the output control section performs display control for displaying the determined health condition in the display apparatus on the user side and displays, based on the determination result of the health condition, one of a first health monitor screen for normal determination when the health condition is determined to be normal, a second health monitor screen for emergency for notifying that an abnormal state occurs in the user when the health condition is determined to be abnormal, and a third health monitor screen for non-normal determination when the health condition is determined to be non-normal, in the display apparatus on the user side.

6. The life watching and support apparatus according to claim 5, wherein, when the health condition is determined to be abnormal, the output control section displays the second health monitor screen including the physiological information associated with the health condition determined to be abnormal, preset contact information of the watcher side, and map information representing an area including a house of the user, in the display apparatus on the user side.

7. The life watching and support apparatus according to any one of claims 1 to 6, wherein, when the health condition is determined to be abnormal, the output control section notifies a preset communication terminal apparatus on the watcher side of the abnormal determination.

8. The life watching and support apparatus according to any one of claims 1 to 7, wherein the display apparatus is a digital television apparatus having a communication function.

9. A program executed by a computer connected to a living behavior acquirement apparatus configured to acquire behavior information of a user, connected to a physiological information acquirement apparatus configured to acquire physiological information of the user, and connected to a display apparatus on a watcher side watching the user, the program comprising:
a first function of estimating a living behavior situation of the user based on the behavior information of the user;
a second function of determining a health condition of the user based on the estimated living behavior situation and the physiological information; and
a third function of displaying a watching screen including the determined health condition in the display apparatus on the watcher side depending on the health condition.

10. A life watching and support system comprising:
a living behavior acquirement apparatus configured to acquire behavior information of a user;
a physiological information acquirement apparatus configured to acquire physiological information of the user;
a display apparatus on a watcher side watching the user; and
a life watching and support apparatus connected to the living behavior acquirement apparatus, the physiological information acquirement apparatus, and the display apparatus on the watcher side,
wherein the life watching and support apparatus includes:
a behavior estimation section configured to estimate a living behavior situation of the user based on the behavior information of the user;
a health condition determination section configured to determine a health condition of the user based on the estimated living behavior situation and the physiological information; and
an output control section configured to display a watching screen including the determined health condition in the display apparatus on the watcher side depending on the health condition.
